# DEMANDE DE BREVET EUROPEEN

(11) **EP 4 345 498 A1**
(43) Date de publication de la demande: **03.04.2024**
(21) Numéro de dépôt: 23194497.6
(22) Date de dépôt: 31.08.2023
(51) Int. Cl.: G01S 7/52, B06B 1/02, G01S 15/89

(54) **PROCÉDÉ POUR PILOTER UN DISPOSITIF ÉMETTEUR**

(30) Priorité: 30.09.2022 FR 2210030
(71) Demandeur: SUPERSONIC IMAGINE, 13290 Aix-en-Provence (FR)
(72) Inventeur: Cordonnier, Hubert, 13290 Aix-en-Provence (FR); Laville, Vincent, 13857 Aix-en-Provence (FR); Martin, Jean-Alain, 13290 Aix-en-Provence (FR); Martinez, Julien, 13290 Aix-en-Provence (FR); Roux, Guillaume, 13290 Aix-en-Provence (FR)
(74) Mandataire: RVDB Nantes

(57) **Abrégé**

La présente divulgation concerne un procédé pour piloter un dispositif émetteur, dans lequel le dispositif émetteur fonctionne avec une fréquences cible (f0). Le procédé comprend : piloter le dispositif émetteur par un signal électrique comprenant différentes fréquences de pilotage (f1, f2) sélectionnées en fonction de la fréquence cible (f0).

## Description

### Technique antérieure

Il est connu d'utiliser un dispositif émetteur, tel qu'un dispositif transducteur comprenant une pluralité d'éléments transducteurs (par exemple disposés en réseau) à des fins de communication, d'imagerie ou de balayage, par exemple dans le domaine de l'imagerie médicale, du radar, du sonar, de la sismologie, des communications sans fil, de la radioastronomie, de l'acoustique et de la biomédecine. Un exemple comprend l'imagerie par ultrasons.

A cet effet, le dispositif émetteur peut fonctionner ou être piloté avec un signal électrique. Conventionnellement, ledit signal électrique a une fréquence prédéfinie. Cette fréquence peut par exemple être choisie en fonction de l'utilisation du dispositif émetteur.

Par exemple, l'imagerie ultrasonore peut avoir pour but d'estimer la réflectivité d'un milieu. Ainsi, la fréquence prédéfinie peut être choisie en fonction des caractéristiques du milieu (par exemple un tissu humain).

Dans un procédé conventionnel d'imagerie par ultrasons, on peut utiliser un dispositif transducteur d'ultrasons (également appelé sonde à ultrasons) avec un ensemble d'éléments transducteurs d'ultrasons. Dans ce procédé, un ou plusieurs transducteurs sont utilisés pour convertir l'énergie électrique en ondes ultrasonores. En particulier, les transducteurs peuvent émettre un ou successivement plusieurs faisceaux ultrasonores dans un milieu, ce qui correspond à une opération d'émission. Ensuite, dans une opération de réception, un ensemble de signaux d'écho rétrodiffusés sont reçus du milieu par le même ensemble ou un autre ensemble d'éléments transducteurs. En particulier, chacun des éléments transducteurs convertit un signal d'écho reçu en un signal électrique, par exemple. Le signal peut ensuite être traité par le système à ultrasons ou par tout système associé (dédié). Par exemple, ils peuvent être amplifiés, filtrés, numérisés et/ou une opération de conditionnement du signal peut être effectuée. Les éléments transducteurs peuvent être disposés comme une ligne de transducteurs ou comme un réseau de transducteurs ou toute autre configuration.

En général, lorsqu'un dispositif émetteur fonctionne avec un signal électrique, le dispositif émet des ondes électromagnétiques (EM). Le dispositif émetteur peut donc également être appelé dispositif émetteur EM.

Il existe différentes normes qui limitent le niveau autorisé d'émissions électromagnétiques des appareils électriques, par exemple les normes IEC 60601-1-2, NF EN 55011 / CISPR 11 et IEC 61000-4-3.

En particulier, la norme CISPR 11 (cf. par exemple l'édition 6.2 2019-01) définit les limites autorisées (c'est-à-dire les valeurs quasi-peak/quasi-crêtes de l'émission électromagnétique) pour différentes plages de fréquences. En conséquence, l'énergie d'un champ électrique causé par un dispositif ne doit pas dépasser ces limites. Par exemple, le tableau 6 de la norme CISPR 11 concerne les limites de perturbation par rayonnement électromagnétique pour les appareils de classe A groupe 1, mesurées sur un site d'essai. De plus, le tableau 2 de la norme CISPR 11 concerne les limites de tension perturbatrice pour les appareils de classe A groupe 1, mesurées sur un site d'essai. Un dispositif médical, comme par exemple une sonde à ultrason, est typiquement de classe A, car il concerne un appareil non-domestique.

En outre, IEC 61000-4-3 / EN 61000-4-3 concerne une norme de compatibilité électromagnétique (CEM), et notamment la partie 4-3 adresse des techniques d'essai et de mesure, et l'essai d'immunité aux rayonnés aux fréquences radioélectriques.

### Exposé de la divulgation

Le procédé et le système décrits dans le présent document concernent des technologies d'optimisation du fonctionnement ou pilotage d'un dispositif émetteur qui peuvent améliorer les performances et/ou la qualité du fonctionnement, malgré un niveau limité d'émissions électromagnétiques causées par le dispositif émetteur.

En particulier, il peut être souhaitable de limiter le niveau des émissions électromagnétiques pour respecter une norme. En outre, il peut être souhaitable de limiter le niveau des émissions électromagnétiques sans avoir d'impact sur les performances de fonctionnement souhaitées du dispositif émetteur.

Par exemple, dans le cas où le dispositif émetteur est une sonde à ultrasons, il peut être souhaitable que la performance et/ou énergie acoustique (et/ou la puissance acoustique) puisse être optimisée et/ou augmentée malgré un niveau limité d'émissions électromagnétiques.

Par conséquent, un procédé pour piloter un dispositif émetteur est fourni selon un exemple de réalisation particulier de la divulgation. Le procédé comprend : piloter le dispositif émetteur par un signal électrique comprenant différentes fréquences de pilotage sélectionnées en fonction de la fréquence cible.

En fournissant un tel procédé, il devient possible d'améliorer les performances et/ou la qualité du fonctionnement, malgré un niveau limité d'émissions électromagnétiques causées par le dispositif émetteur.

Le procédé nécessite simplement une modification du signal électrique. Donc, le procédé peut être facilement mise en oeuvre dans les systèmes et les dispositifs émetteur existants, sans nécessiter de dispositifs supplémentaires et/ou des modifications structurelles du système ou du dispositif. Les coûts de mise en oeuvre du procédé peuvent donc être réduits.

En outre, l'augmentation de la performance de fonctionnement peut être obtenue dans n'importe quel type de mode de fonctionnement. Des exemples de différents modes de fonctionnement d'un dispositif émetteur sous la forme d'une sonde à ultrasons peuvent comprendre un mode B (c'est-à-dire un mode de luminosité), un mode Doppler ou un mode SWE (c'est-à-dire un mode d'élastographie par ondes de cisaillement). En d'autres termes, il n'est pas nécessaire de changer ou de modifier le mode de fonctionnement spécifique pour obtenir l'augmentation des performances, mais l'augmentation est obtenue indépendamment de la sélection du mode de fonctionnement.

Le procédé selon la divulgation peut comporter d'autres caractéristiques qui peuvent être prises séparément ou en combinaison, notamment parmi les modes de réalisation qui suivent.

Selon un exemple de réalisation particulier, la fréquence cible correspond à la fréquence d'un signal électrique qui serait conventionnellement utilisé pour piloter le dispositif émetteur.

Selon un exemple de réalisation particulier, au moins l'une des fréquences de pilotage du signal électrique est différente de la fréquence cible. En conséquence, les fréquences de pilotage peuvent être différentes les unes des autres.

Selon un exemple de réalisation particulier, les fréquences de pilotage sont suffisamment différentes et/ou éloignées l'une de l'autre de telle sorte que : leur champ spectral additionné ne dépasse pas le champ spectral de l'une quelconque des fréquences de pilotage individuelles. Par conséquent, le maximum défini par le champ spectral total ne dépasse pas les maxima des champs spectraux des différentes fréquences.

Le champ spectral peut être (ou peut comprendre) un champ électrique et/ou électro-magnétique.

Le champ spectral additionné peut constituer des fréquences et de leurs harmoniques. Par exemple, il est possible de choisir le champ spectral additionné tel qu'il ne dépasse pas le champ spectral de l'une quelconque des fréquences de pilotage individuelles ou de leurs harmoniques.

Selon un exemple de réalisation particulier, les fréquences de pilotage sont sélectionnées de telle sorte que la fréquence cible puisse être atteinte par une combinaison de la pluralité de fréquences de pilotage.

En conséquence, les différentes fréquences de pilotage peuvent être utilisées à la place de la fréquence cible, afin de piloter le dispositif émetteur. En conséquence, le dispositif émetteur fonctionne comme s'il était piloté avec un signal électrique ayant la fréquence cible.

Selon un exemple de réalisation particulier, le signal électrique peut être composé de formes d'onde successives telles que chaque forme d'onde a pour fréquences une des fréquences de pilotage.

Une forme d'onde au sens de la présente divulgation peut être un élément du signal dans le domaine temporel. En conséquence, une forme d'onde peut également être appelée composante temporelle.

Selon un exemple de réalisation particulier, les formes d'onde sont répétées (par exemple périodiquement), et/ou les formes d'onde sont alternées entre elles, de sorte que chaque forme d'onde soit partiellement interrompue.

Selon un exemple de réalisation particulier, chaque forme d'onde est pondérée par une valeur d'impédance et/ou de courant en fonction du signal électrique cible.

Selon un exemple de réalisation particulier, les formes d'onde forment une séquence temporelle prédéfinie de N1 impulsions de fréquences de pilotage f1, suivi de N2 impulsions de fréquences de pilotage f2, ..., suivi de Nn impulsions de fréquences de pilotage fn,
dans lequel N1, N2, ..., Nn sont des nombres entiers supérieurs ou égaux à 1.

Cependant, les formes d'onde peuvent aussi former une séquence temporelle aléatoire de pulsations de fréquences de pilotage.

Selon un exemple de réalisation particulier, les formes d'onde et/ou les impulsions de fréquences de pilotage sont configurées pour stimuler un élément piézoélectrique (ou un autre type d'élément transducteur) d'une sonde à ultrasons (ou d'un autre type de dispositif transducteur).

Selon un exemple de réalisation particulier, le dispositif est piloté (ou est configuré pour être piloté) par un signal électrique ayant la fréquence cible, dans lequel les formes d'onde sont sélectionnées, de telle sorte que leur ensemble puisse atteindre (et/ou approcher et/ou approximer) le signal électrique cible. Par conséquent, le dispositif émetteur peut finalement fonctionner avec un signal électrique adapté au dispositif compte tenu de la fréquence cible du dispositif.

Selon un exemple de réalisation particulier, les formes d'onde sont sélectionnées de telle sorte qu'une énergie moyenne, dite « Q-Peak », de chaque forme d'onde soit inférieure à l'énergie moyenne du signal électrique cible.

Donc, la séquence temporelle déterministe (ou prédéfinie) peut par exemple comprendre N1 impulsions de fréquences f1, suivi de N2 impulsions de fréquences f2, ..., suivi de Nn impulsions de fréquences fn. N1, N2, ..., Nn peuvent être des nombres entiers supérieurs ou égaux à 1 et pondérés par les valeurs des impédances ou des courants (par exemple mesurés avant, optionnellement de manière automatique) du dispositif émetteur aux fréquences f1, f2, ... , fn, afin que l'énergie moyenne Q-Peak soit identique à chacune de ces fréquences.

Selon un exemple de réalisation particulier, le procédé est un procédé médical et/ou à ultrasons, et/ou le dispositif est une sonde médicale et/ou à ultrasons.

Cependant, le dispositif émetteur peut généralement être tout dispositif qui fonctionne grâce à un signal électrique et/ou qui émet des ondes électromagnétiques.

Selon un exemple de réalisation particulier, le dispositif émetteur est piloté (ou est configuré pour être piloté) par une pluralité de signaux électriques, dont chacun comprend différentes fréquences de pilotage sélectionnées en fonction de la fréquence cible respective. Par exemple, dans le cas où le dispositif émetteur est un dispositif transducteur avec une pluralité d'éléments transducteurs, chaque élément transducteur peut fonctionner ou être piloté avec un signal électrique respectif. Par conséquent, le procédé peut fournir une pluralité de signaux électriques pour une pluralité respective d'éléments transducteurs.

En conséquence, chacun de la pluralité d'éléments transducteurs du dispositif émetteur peut avoir une fréquence cible respective (par exemple la même fréquence cible).

Selon un exemple de réalisation particulier, le dispositif émet (ou est configuré pour émettre) une onde pour traiter et/ou observer le milieu.

Selon un exemple de réalisation particulier, le dispositif émet (ou est configuré pour émettre) une onde électro-magnétique lorsqu'il est piloté.

La présente divulgation se rapporte également un programme d'ordinateur comprenant des instructions qui, lorsque le programme est exécuté par un ordinateur, conduisent celui-ci à mettre en oeuvre le procédé selon l'un quelconque des aspects précédents.

La présente divulgation selon un mode de réalisation particulier se rapporte également un système pour piloter un dispositif émetteur. Le dispositif émetteur fonctionne avec une fréquences cible. Le système est configuré pour : piloter le dispositif émetteur par un signal électrique comprenant différentes fréquences de pilotage sélectionnées en fonction de la fréquence cible.

Le système peut avoir des fonctionnalités qui correspondent aux opérations du procédé selon la présente divulgation.

Les caractéristiques et avantages de la divulgation apparaitront à la lecture de la description qui va suivre, donnée uniquement à titre d'exemple non limitatif, et faite en référence aux figures annexées. Notamment, les exemples illustrés dans les figures peuvent être combinés sauf incohérence notoire.

### Brève description des figures

D'autres caractéristiques et avantages de la présente divulgation ressortiront de la description des exemples de réalisation particuliers et non limitatifs de la présente divulgation ci-après, en référence aux figures 1 à 6 annexées, sur lesquelles :
[Fig. 1] montre un dessin schématique d'un système de pilotage d'un dispositif émetteur selon des exemples de la présente divulgation.
[Fig. 2] montre un dessin schématique d'un système d'imagerie ultrasonore selon des exemples de la présente divulgation.
[Fig. 3] montre schématiquement un diagramme temporel et un diagramme spectral illustrant un pilotage conventionnel d'un dispositif émetteur.
[Fig. 4] montre schématiquement un diagramme temporel et un diagramme spectral illustrant un pilotage d'un dispositif émetteur selon des exemples de la présente divulgation.
[Fig. 5] montre schématiquement un premier exemple de relation entre des fréquences de pilotage choisies et une distribution spectrale d'énergie résultante du champ électrique provoqué par le dispositif émetteur.
[Fig. 6] montre schématiquement un deuxième exemple de relation entre des fréquences de pilotage choisies et une distribution spectrale d'énergie résultante du champ électrique provoqué par le dispositif émetteur selon des exemples de la présente divulgation.

### Description des modes de réalisation

Sur les différentes figures, fournies à titre d'illustration, les mêmes références numériques désignent des éléments identiques ou similaires.

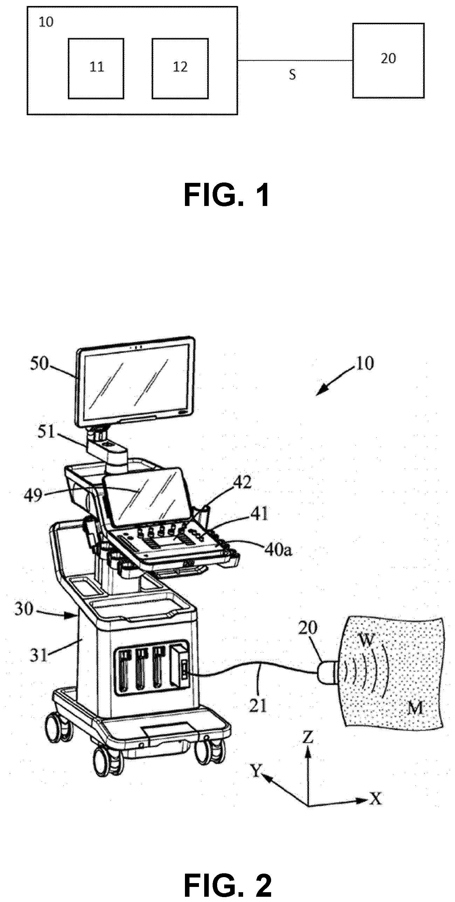

La figure 1 montre un dessin schématique d'un système 10 pour piloter un dispositif émetteur 20 selon des exemples de la présente divulgation.

Le système 10 peut également comprendre le dispositif émetteur. En outre, le dispositif peut être externe au système. Par exemple, le dispositif 20 peut être connecté au système par un câble ou peut communiquer sans fil. Dans ce dernier cas, le dispositif 20 peut par exemple comprendre une batterie et recevoir un signal de communication du système qui représente le signal électrique (par exemple les fréquences de pilotage et/ou toute information comprise dans le signal électrique). Le dispositif 20 peut alors générer le signal électrique en interne.

Le système peut être un système conventionnel et/ou le dispositif émetteur 20 peut être un dispositif émetteur conventionnel. En conséquence, la seule différence selon la présente divulgation peut être la manière dont le dispositif émetteur est exploité par le système. Cependant, il peut ne pas y avoir de différences structurelles entre le système et le dispositif émetteur selon la présente divulgation et des systèmes et dispositifs émetteur connus.

Le système peut être stationnaire ou mobile. Le dispositif émetteur peut également être stationnaire ou mobile. Par exemple, le système peut être un système fixe (par exemple comprenant une unité de traitement et un dispositif d'affichage, comme décrit ci-dessous) et le dispositif émetteur peut être mobile (par exemple un dispositif capteur, un dispositif de mesure, ou plus particulièrement une sonde). Toutefois, il est également possible que le dispositif émetteur soit intégré au système, et que le système soit un système mobile. Par exemple, le système peut être configuré pour être piloté de manière autonome, par exemple grâce à une batterie incluse. D'autres exemples sont décrits ci-dessous.

Le dispositif émetteur peut être configuré pour fonctionner avec un signal électrique comprenant une fréquence f0. En conséquence, le dispositif émetteur 20 peut conventionnellement fonctionner avec la fréquence f0.

Le dispositif émetteur 20 provoque typiquement une émission électromagnétique EM du fait de son pilotage. Ladite émission électromagnétique (EM) peut nécessiter d'être inférieure à un seuil EM maximal, qui est par exemple défini par une norme.

Le système est configuré pour piloter le dispositif émetteur 20 avec un signal électrique S. Le signal électrique S comprend différentes fréquences de pilotage f1, f2. Les fréquences de pilotage f1, f2 sont sélectionnées en fonction de la fréquence f0. Pour cette raison, la fréquence f0 peut également être appelée fréquence cible.

Par exemple, le système peut comprendre une unité de commande de puissance 12 configurée pour générer le signal électrique S. En conséquence, ladite unité de commande de puissance 12 peut être configurée pour moduler un signal électrique de sorte qu'il ait différentes fréquences de pilotage f1, f2 selon la présente divulgation. L'unité de commande de puissance 12 peut par exemple être ou comprendre un pulseur électronique ou simplement « pulseur » (c'est à dire un générateur d'impulsions électroniques). Le système peut également comprendre une unité de traitement 11. L'unité de traitement peut par exemple être configurée pour commander l'unité de commande de puissance 12. Par exemple, l'unité de traitement 11 peut calculer les fréquences de pilotage f1, f2 en fonction de la fréquence cible f0.

L'unité de traitement peut en outre être configurée pour commander le dispositif émetteur, par exemple en commandant le signal électrique S.

Selon d'autres exemples, le système 10 peut comprendre au moins une unité de traitement (ou processeur) 11 et en plus une mémoire (non représentée). Dans des exemples, l'unité de traitement et de mémoire peut être incorporée dans le système tel que représenté sur la figure 1 ou peut être un ordinateur ou un dispositif informatique lié de manière communicative à celui-ci. Selon la configuration exacte et le type de dispositif informatique, la mémoire (qui stocke les instructions permettant d'évaluer les données ultrasonores ou d'exécuter les procédés décrits dans le présent document) peut être volatile (telle que la RAM), non volatile (telle que la RAM, la mémoire flash, etc.) ou une combinaison des deux. En outre, le système 10 peut également comprendre des dispositifs de stockage (amovibles et/ou non amovibles), y compris, mais sans s'y limiter, des disques magnétiques ou optiques ou des bandes. De même, le système 10 peut également comporter un ou plusieurs dispositifs d'entrée tels qu'un clavier, une souris, un stylo, une entrée vocale, etc. et/ou un ou plusieurs dispositifs de sortie tels qu'un écran, des haut-parleurs, une imprimante, etc. L'environnement peut également comprendre une ou plusieurs connexions de communication, telles que LAN, WAN, point à point, etc. Dans certains modes de réalisation, les connexions peuvent être utilisées pour établir des communications point à point, des communications orientées connexion, des communications sans connexion, etc.

Le système 10 peut en outre comprendre au moins une certaine forme de support lisible par ordinateur. Les supports lisibles par ordinateur peuvent être n'importe quel support disponible auquel l'unité de traitement (ou le processeur) ou d'autres dispositifs comprenant l'environnement d'exploitation peuvent accéder. À titre d'exemple, et sans limitation, les supports lisibles par ordinateur peuvent comprendre des supports de stockage informatique et des supports de communication. Les supports de stockage informatique comprennent des supports volatils et non volatils, amovibles et non amovibles, mis en oeuvre dans tout procédé ou technologie de stockage d'informations telles que des instructions lisibles par ordinateur, des structures de données, des modules de programme ou d'autres données. Les supports de stockage informatique ne comprennent pas les supports de communication.

Les supports de communication intègrent des instructions lisibles par ordinateur, des structures de données, des modules de programme ou d'autres données dans un signal de données modulé tel qu'une onde porteuse ou un autre mécanisme de transport, et comprennent tout support de fourniture d'informations. L'expression "signal de données modulé" désigne un signal dont une ou plusieurs des caractéristiques sont fixées ou modifiées de manière à coder des informations dans le signal. À titre d'exemple, et sans limitation, les supports de communication comprennent les supports câblés tels qu'un réseau câblé ou une connexion câblée directe, et les supports sans fil tels que les supports acoustiques, RF, infrarouges, micro-ondes et autres supports sans fil. Les combinaisons de l'un quelconque des éléments ci-dessus doivent également être incluses dans le champ d'application des supports lisibles par ordinateur.

Le système 10 peut être un ordinateur unique fonctionnant dans un environnement en réseau utilisant des connexions logiques avec un ou plusieurs ordinateurs distants. L'ordinateur distant peut être un ordinateur personnel, un serveur, un routeur, un PC de réseau, un dispositif homologue ou un autre noeud de réseau commun, et comprend généralement plusieurs ou tous les éléments décrits ci-dessus ainsi que d'autres non mentionnés. Les connexions logiques peuvent inclure toute méthode supportée par les supports de communication disponibles. De tels environnements de mise en réseau sont courants dans les bureaux, les réseaux informatiques d'entreprise, les intranets et l'Internet.

Le système 10 peut être un système médical, par exemple un système à ultrasons. En conséquence, le dispositif émetteur 20 peut être une sonde médicale et/ou à ultrasons.

Par exemple, le système peut être associé à une sonde à ultrasons 20, afin de collecter des données ultrasonores d'un milieu, par exemple des tissus vivants et/ou en particulier des tissus humains d'une personne.

La sonde à ultrasons 20 peut comprendre par exemple un ou plusieurs éléments transducteurs non représentés sur la figure 1), chacun étant configuré pour convertir un signal électrique reçu du système 10 en ondes ultrasonores. Les éléments transducteurs peuvent être configurés pour transmettre (a) les ondes dans le milieu et/ou pour recevoir (b) une pluralité de signaux ultrasonores du milieu, éventuellement en réponse à la transmission (a). Un réseau de transducteurs comprenant une pluralité d'éléments transducteurs peut être utilisé. Le ou les mêmes éléments transducteurs peuvent être utilisés pour émettre une impulsion et recevoir la réponse, ou des éléments transducteurs différents sont utilisés pour l'émission et la réception. Il peut y avoir un ou plusieurs éléments transducteurs d'émission et une pluralité d'éléments transducteurs de réception. Dans une autre variante, un seul élément transducteur peut être utilisé. Les éléments transducteurs peuvent comprendre des cristaux piézoélectriques et/ou d'autres composants qui peuvent être configurés pour générer et/ou enregistrer et/ou recevoir des signaux.

La sonde à ultrasons 20 peut en outre comprendre un dispositif de commande électronique (non représenté sur la figure 1) commandant les éléments transducteurs et acquérant des signaux à partir de ceux-ci. Le dispositif de commande électronique peut faire partie de la sonde. En variante, le dispositif de commande électronique peut être externe à la sonde (par exemple, faire partie de l'unité de commande de puissance 12), ou consister en plusieurs dispositifs qui font partiellement partie de la sonde et sont partiellement externes à celle-ci.

En outre, l'unité de traitement 11 peut être configurée pour recevoir des données du dispositif émetteur 20, traiter des données et/ou envoyer des données à un dispositif externe, comme par exemple, un dispositif d'affichage, un serveur, un ordinateur sur lequel un algorithme d'intelligence artificielle (IA) est exécuté, une station de travail dédiée, ou tout autre dispositif externe.

Toutefois, le système peut être n'importe quel type de système électronique. Par exemple, le système peut également être un autre type de système médical qu'un système d'imagerie par ultrasons. En conséquence, le dispositif émetteur 20 peut être n'importe quel type de dispositif d'imagerie ou de capteur, utilisant d'autres ondes que les ondes ultrasonores (par exemple, des ondes ayant une longueur d'onde différente de la longueur d'onde des ultrasons et/ou des ondes n'étant pas des ondes sonores).

Selon d'autres exemples, le système et/ou le dispositif émetteur est configuré à des fins de communication, d'imagerie ou de balayage, par exemple dans le domaine de l'imagerie médicale, du radar, du sonar, de la sismologie, des communications sans fil, de la radioastronomie, de l'acoustique et de la biomédecine.

Des exemples de systèmes d'imagerie médicale comprennent un système d'imagerie par ultrasons, un système d'imagerie par rayons X (en particulier pour la mammographie) et un système IRM (Imagerie par résonance magnétique).

La figure 2 montre un dessin schématique d'un système d'imagerie ultrasonore 10 selon des exemples de la présente divulgation. Le système d'imagerie par ultrasons 10 peut être un exemple de système de pilotage d'un dispositif émetteur 10, tel que décrit dans le contexte de la figure 1.

Le système d'imagerie par ultrasons 10 peut comprendre :
- une sonde 20 (correspondant par exemple au dispositif d'émetteur 20 de la figure 1),
- une unité de traitement 30 pour traiter une image sur la base des signaux reçus par la sonde (par exemple correspondant à l'unité de traitement 11 de la figure 1),
- un panneau de commande 40 relié à l'unité de traitement, ledit panneau de commande comprenant au moins des boutons 41 et une tablette tactile 42, et
- un écran 50 pour visualiser l'image.

La sonde 20 peut être connectée à l'unité de traitement 30 par un câble 21 ou par une connexion sans fil, et elle est capable d'émettre des ondes ultrasonores W dans un milieu M et de recevoir des ondes ultrasonores W du milieu M, lesdites ondes ultrasonores reçues étant conséquentes ou résultant de réflexions desdites ondes ultrasonores émises sur des particules diffusantes à l'intérieur dudit milieu. La sonde 20 peut être un réseau de transducteurs comprenant une pluralité de transducteurs, chacun convertissant un signal électrique en une vibration et réciproquement. Un transducteur est par exemple un élément piézoélectrique. Le réseau de transducteurs peut comprendre cent transducteurs ou plus. Le réseau de transducteurs est linéaire ou incurvé et est disposé sur une surface extérieure du milieu M de manière à être couplé au milieu et à vibrer et à émettre ou recevoir des ondes ultrasonores W.

L'unité de traitement 30 peut comprendre des dispositifs de réception pour amplifier et/ou filtrer les signaux reçus de la sonde 20, et des convertisseurs (convertisseurs analogiques-numériques et convertisseurs numériques-analogiques) pour transformer les signaux en données représentant le signal. Les données peuvent être stockées dans une mémoire de l'unité de traitement ou traitées directement pour calculer des données traitées intermédiaires (données de formation de faisceau). L'unité de traitement 30 peut utiliser toute méthode de traitement connue pour traiter l'image sur la base des signaux reçus de la sonde, telle que la formation de faisceau. Les données d'image ultrasonore traitées peuvent être :
- une image simple du milieu (image en mode B) généralement en échelle de gris pour visualiser les organes à l'intérieur du milieu, ou
- une image montrant la vitesse ou l'écoulement dans le milieu (image couleur), par exemple utile pour visualiser les vaisseaux sanguins dans le milieu, ou
- une image montrant une caractéristique mécanique du milieu (élasticité), par exemple utile pour identifier des tumeurs à l'intérieur du milieu (par exemple des données d'image d'élastographie par ondes de cisaillement (ShearWave^{™} Elastographiy, SWE) comme décrit ci-dessous).

L'écran d'affichage 50 peut être un écran de visualisation de l'image traitée par l'unité de traitement 30. L'écran d'affichage 50 peut également visualiser d'autres informations telles que les échelles utilisées dans l'image, ou des informations de configuration pour le traitement ou toute information telle que des informations d'aide ou une aide gestuelle contextuelle pour le pavé tactile 42.

L'écran d'affichage peut être articulé sur un bras de support 51 pour un meilleur positionnement de l'utilisateur. L'écran d'affichage est généralement un écran de grande taille (au moins 20 pouces) pour une meilleure visualisation des images par l'utilisateur.

Le panneau de commande 40a est par exemple une partie du boîtier du système 31, ladite partie comprenant un boîtier de panneau ayant une surface sensiblement plane inclinée vers l'utilisateur pour être manipulée par une main dudit utilisateur. Le panneau de commande 40a peut être déplacé par un cintre vers le haut et vers le bas pour être adapté à la taille de l'utilisateur, et peut éventuellement être déplacé vers l'avant et vers l'arrière pour être adapté à la position de l'utilisateur. Comme on le voit sur la figure 1, le panneau de commande 40a peut comprendre un écran d'affichage du panneau de commande 49 pour visualiser plusieurs informations de configuration. Cet écran d'affichage du panneau de commande 49 peut également être articulé sur le boîtier du panneau de commande 48 pour être incliné dans une inclinaison différente de celle de la surface du panneau de commande.

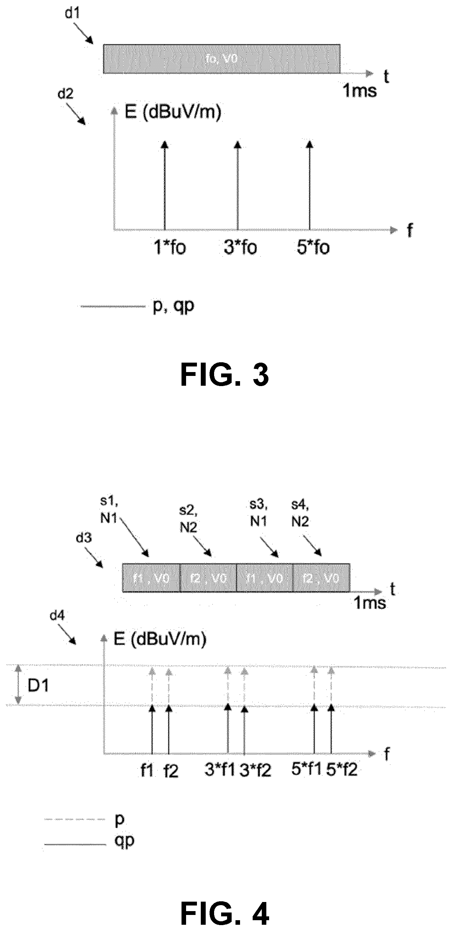

La figure 3 montre schématiquement un diagramme temporel et un diagramme spectral illustrant un pilotage conventionnel d'un dispositif émetteur. En particulier, le diagramme d1 montre un diagramme temporel d'un signal électrique conventionnel pour piloter un dispositif émetteur, et le diagramme d2 montre le diagramme spectral respectif du signal électrique conventionnel.

Comme le montre le diagramme temporel d1, un dispositif émetteur peut fonctionner de manière conventionnelle avec un signal électrique comprenant une seule fréquence f0 pendant toute la durée du pilotage. La fréquence peut être choisie en fonction de l'objectif du dispositif émetteur. Par exemple, dans le cas où le dispositif émetteur est une sonde à ultrasons pour l'imagerie d'un milieu, la fréquence f0 peut être choisie pour correspondre aux caractéristiques du milieu. Par exemple, la fréquence peut être f0 = 5MHz.

Par exemple, une fréquence d'environ 5 Mhz (ou toute valeur comprise entre par exemple 1 et 20 MHz) peut être choisie pour générer des ondes ultrasonores pour l'imagerie d'un tissu humain ou animal. Cette valeur peut être avantageuse pour cette application, car l'atténuation causée par le tissu dans cette gamme de fréquences est relativement faible et, en même temps, les signaux des ondes ultrasonores dans cette gamme peuvent être facilement générés par un élément piézoélectrique d'un élément transducteur à ultrasons. Ainsi, les ondes ultrasonores à fréquences basses (dans la bande de fréquence utile des éléments piézoélectriques) peuvent pénétrer plus profondément dans les tissus. Le choix d'une telle fréquence (5MHz par exemple) peut donc être un compromis entre la profondeur de pénétration et le rendement électro-acoustique de l'élément piézoélectrique.

Dans l'exemple du diagramme d1, le temps de pilotage est de 1ms. Toutefois, le temps de pilotage peut également être plus long ou plus court. Le temps de pilotage peut par exemple correspondre au temps d'émission d'une impulsion ultrasonore (émise par le dispositif émetteur). De plus, le signal électrique peut avoir une tension de, par exemple, V0 = 50V.

Le diagramme spectral correspondant d2 peut indiquer le champ électrique (dBuV/m) (et par conséquent la puissance et/ou énergie) émis par le dispositif émetteur en fonction de la fréquence (f). Comme présenté sur le diagramme spectral d2, le signal électrique peut comprendre une fréquence f0 et des harmoniques 3*f0, 5*f0, etc. Une puissance et/ou énergie instantanée p (c'est-à-dire une puissance crête) et une puissance et/ou énergie moyenne qp (c'est-à-dire une "quasi-crête") sur la durée totale de pilotage sont similaires. Il est à noter que dans la présente divulgation, "énergie moyenne " et "puissance" peuvent être utilisés de manière interchangeable, car les paramètres peuvent correspondre l'un à l'autre, en tenant compte d'un temps particulier (par exemple une impulsion de 1 ms).

Par exemple, ce temps particulier à prendre en compte peut être de 1 ms dans les exemples des figures 3 et 4. Ainsi, dans l'exemple de la figure 4, l'énergie moyenne émis par le dispositif émetteur dans une seule fréquence de pilotage (par exemple f1) est réduite, car le temps total des formes d'onde respectives (par exemple S1 et S3) est plus court que le temps total pris en compte (par exemple 1ms).

L'émission électromagnétique causée par le dispositif émetteur en raison de son pilotage peut être définie par la puissance et/ou énergie moyenne. En conséquence, une norme qui régit le niveau autorisé d'émission électromagnétique peut exiger de limiter la puissance et/ou énergie moyenne. Par conséquent, dans un procédé de pilotage conventionnelle telle qu'illustrée à la figure 3, la puissance et/ou énergie instantanée doit également être limitée, respectivement.

Dans le cas de l'exemple mentionné où une sonde à ultrasons fonctionne avec un signal électrique ayant une fréquence de f0 = 5 MHz, et où l'on considère également des harmoniques de 25 MHz, la quasi-crête de rayonnement électromagnétique autorisée peut être limitée à 40 dB (voir par exemple le tableau 6 de la norme CISPR 11 citée ci-dessus). Au-delà de 230 MHz, la quasi-crête autorisée peut être limitée à 47 dB. Le rayonnement électromagnétique d'une sonde à ultrasons doit donc se situer en dessous de ces limites supérieures pour satisfaire en particulier ces normes.

La figure 4 montre schématiquement un diagramme temporel et un diagramme spectral illustrant un pilotage d'un dispositif émetteur selon des exemples de la présente divulgation.

Les diagrammes d3 et d4 correspondent principalement aux diagrammes d1 et d2 de la figure 3. Cependant, le procédé selon la présente divulgation est appliqué pour piloter le dispositif émetteur. En particulier, le diagramme d3 montre un diagramme temporel d'un signal électrique pour piloter un dispositif émetteur selon des exemples de la présente divulgation, et le diagramme d4 montre le diagramme spectral respectif du signal électrique selon des exemples de la présente divulgation.

Comme le montre le diagramme temporel d3, un dispositif émetteur est piloté avec un signal électrique comprenant différentes fréquences de pilotage f1, f2. On peut également utiliser plus de deux fréquences de pilotage. Les fréquences de pilotage sont sélectionnées en fonction de la fréquence cible f0. La fréquence cible peut être la fréquence f0 utilisée dans l'exemple de la figure 3. Par exemple, la fréquence peut être f0 = 5MHz. En conséquence, la fréquence f1 peut être par exemple f1 = 4,5 MHz et/ou la fréquence f2 peut être par exemple f2 = 5,5 MHz. Le signal électrique peut avoir une tension de par exemple de V0 = 50V.

Généralement, au moins une des fréquences de pilotage f1, f2 du signal électrique peut être différente de la fréquence cible. Les fréquences de pilotage sont préférablement sélectionnées de telle sorte que la fréquence cible puisse être atteinte par une combinaison de la pluralité de fréquences de pilotage.

En conséquence, les différentes fréquences de pilotage peuvent être utilisées à la place de la fréquence cible, afin de piloter le dispositif émetteur. En conséquence, le dispositif émetteur fonctionne comme s'il était piloté avec un signal électrique ayant la fréquence cible.

Le signal électrique peut être composé des formes d'onde successives S1, S2, S3, S4..., telles que chaque forme d'onde a pour fréquences une des fréquences de pilotage f1, f2.

La forme d'onde peut être un élément du signal dans le domaine temporel. En conséquence, la forme d'onde peut également être appelée composante temporelle.

Comme indiqué dans l'exemple de la figure 4, les formes d'onde relatives aux fréquences peuvent être répétées, par exemple périodiquement. En outre, les formes d'onde peuvent être alternées entre elles, de sorte que chaque forme d'onde soit partiellement interrompue.

Notamment, les formes d'onde peuvent former une séquence temporelle prédéfinie de N1 impulsions de fréquences de pilotage f1, suivi de N2 impulsions de fréquences de pilotage f2, ..., suivi de Nn impulsions de fréquences de pilotage fn, N1, N2, ..., Nn étant des nombres entiers supérieurs ou égaux à 1.

Cependant, les formes d'onde peuvent former aussi une séquence temporelle aléatoire de pulsations de fréquences de pilotage.

Chaque forme d'onde peut être pondérée par une valeur d'impédance et/ou de courant en fonction du signal électrique cible.

Dans l'exemple du diagramme d3, le temps de pilotage est de 1ms. Toutefois, le temps de pilotage peut également être plus long ou plus court. Le temps de pilotage peut par exemple correspondre au temps d'émission d'une impulsion ultrasonore (émise par le dispositif émetteur).

Comme le montre le diagramme spectral correspondant d4, le signal électrique peut comprendre les fréquences f1, f2 et les harmoniques 3*f1, 3*f2, 5*f1, 5*f2, etc. La puissance et/ou énergie moyenne qp (c'est-à-dire une "quasi-crête") sur le temps de pilotage complet (par exemple 1ms) peut être inférieure à la puissance et/ou énergie instantanée p (c'est-à-dire une crête de puissance). Cette différence peut être due aux intervalles de temps (indiqués dans le diagramme d3), pendant lesquels une forme d'onde spécifique (par exemple de fréquence f1) n'est pas utilisée et donc pas émise par le dispositif émetteur.

Cette différence peut s'appliquer aux fréquences f1 et f2, et éventuellement aussi à une ou plusieurs de leurs harmoniques.

Notamment, les formes d'onde (illustrée dans le diagramme d3) peuvent être sélectionnées de telle sorte qu'une puissance et/ou énergie moyenne (Q-Peak) de chaque forme d'onde soit inférieure à la puissance et/ou énergie moyenne du signal électrique cible.

En conséquence, étant donné que l'émission électromagnétique causée par le dispositif émetteur peut être définie par la puissance et/ou énergie moyenne, l'émission électromagnétique peut être réduite, même si la puissance et/ou énergie instantanée est plus élevée.

Par exemple, une norme qui régit le niveau autorisé d'émission électromagnétique peut être respectée en raison de l'énergie et/ou puissance moyenne limitée.

En conséquence, selon la présente divulgation, la puissance et/ou énergie instantanée n'a pas besoin d'être limitée pour être inférieure à cette limite. Par conséquent, les performances du dispositif émetteur peuvent être avantageusement augmentées, tout en respectant les limitations requises par la ou les normes

Dans un exemple, la différence D1 entre la puissance et/ou énergie instantanée et la puissance et/ou énergie moyenne peut être D1 = 20*log(2) = 6dB.

Un mode de fonctionnement d'un dispositif émetteur sous la forme d'une sonde à ultrasons peut comprendre par exemple un mode ShearWave^{™}, dit SWE (c'est-à-dire un mode d'élastographie par ondes de cisaillement). Ce mode comprend une étape d'excitation au cours de laquelle on génère une onde élastique de cisaillement dans le milieu, comme décrit par exemple dans le brevet EP 1 546 757 B1 de la même demanderesse. Au cours de l'étape d'excitation (a), l'onde élastique de cisaillement est générée grâce à émission d'au moins une onde ultrasonore focalisée (« PUSH ») suffisament puissante dans le milieu. Par exemple, une onde ultrasonore focalisée (« PUSH ») peut correspondre à une forme d'onde selon la présente divulgation.

Les séquences d'émissions utilisées pour le PUSH peuvent être constitués de milliers de périodes de signal carré à une fréquence donnée. Selon la présente divulgation il est avantageusement possible d'utiliser alternativement une période signal carré à une 1ère fréquence f1 et une période de signal carré à une 2nde fréquence f2, ce qui permet de réduire les pics du spectre fréquentiel lors des mesures d'émission électromagnétique (par exemple de la compatibilité électromagnétique (CEM) et d'interférence électromagnétique (EMI)).

Une version élaborée de la présente divulgation peut utiliser un nombre quelconque de fréquences centrales, séquencées dans le temps de manière aléatoire, avec une pondération temporelle et/ou en amplitude (tension, rapport cyclique...), dans n'importe quel mode d'imagerie et pour n'importe quelle sonde à ultrasons, ce qui permet d'élargir le spectre fréquentiel émis lors des mesures d'émissions électromagnétiques. La séquence aléatoire de fréquences permet de réduire les bruits électriques intrinsèques au système et donc permettent d'améliorer la qualité image. La pondération permet d'optimiser l'amplitude des pics du spectre fréquentiel émis lors des mesures d'émissions électromagnétiques, de manière à conserver une marge par rapport aux limites d'une norme, et ce dans toute la bande fréquentielle.

Par conséquent, une première variante selon un exemple de la présente divulgation peut utiliser un nombre quelconque de fréquences centrales, séquencées dans le temps de manière déterministe, dans n'importe quel mode d'imagerie et pour n'importe quelle sonde à ultrasons, ce qui permet de réduire les pics du spectre fréquentiel lors des mesures dites CEM/EMI de validation des systèmes vis-à-vis les normes

Une deuxième variante selon un exemple de la présente divulgation peut utiliser un nombre quelconque de fréquences centrales, séquencées dans le temps de manière aléatoire, dans n'importe quel mode d'imagerie et pour n'importe quelle sonde à ultrasons, ce qui permet de réduire les pics du spectre fréquentiel lors des mesures CEM/EMI. La séquence aléatoire de fréquence permet de réduire les bruits électriques intrinsèques au système et donc permettent d'améliorer la la qualité image.

Une troisième variante selon un exemple de la présente divulgation peut utiliser un nombre quelconque de fréquences centrales, séquencées dans le temps, avec une pondération temporelle et/ou en amplitude (tension, rapport cyclique...), dans n'importe quel mode d'imagerie et pour n'importe quelle sonde à ultrasons, ce qui permet d'élargir le spectre fréquentiel émis lors des mesures CEM/EMI. La pondération permet d'optimiser l'amplitude des pics du spectre fréquentiel émis lors des mesures CEM/EMI, de manière à conserver une marge par rapport aux limites des normes CEM/EMI, dans toute la bande fréquentielle.

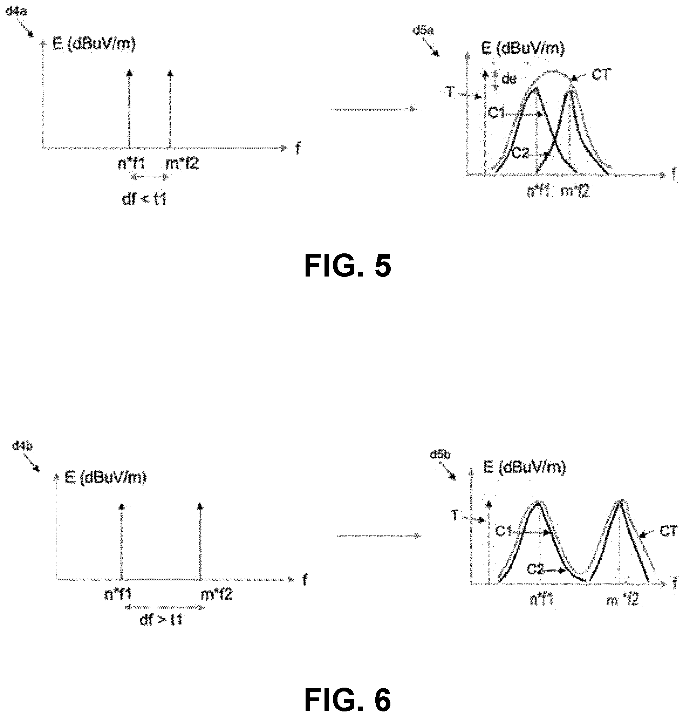

La figure 5 montre schématiquement un premier exemple de relation entre des fréquences de pilotage choisies et une distribution spectrale d'énergie résultante du champ électrique (et donc de l'émission électromagnétique) provoqué par le dispositif émetteur.

Le schéma d4a correspond principalement au schéma d3 de la figure 4. En conséquence, le signal électrique peut comprendre les fréquences f1, f2 et les harmoniques 3*f1, 3*f2, 5*f1, 5*f2, etc. Pour simplifier l'illustration schématique de la figure 5, les fréquences sont indiquées par n*f1 et m*f2, où n = 1, 2, 3, ... et/ou m = 1, 2, 3, ....

Le diagramme d5a montre schématiquement une distribution spectrale d'énergie résultante de l'émission électromagnétique du dispositif émetteur. En particulier, le schéma d5a montre le champ électrique total (c'est-à-dire le champ sommé) CT résultant de la somme des champs électriques C1 de fréquence n*f1 et C2 de m*f2.

Comme le montre le schéma d5a, la différence entre les fréquences n*f1 et m*f2 peut influencer le maximum T du champ total (c'est-à-dire le champ additionné) CT et donc l'énergie et/ou puissance moyenne respective.

Dans l'exemple de la figure 5, la distance df entre les fréquences n*f1 et m*f2 peut être inférieure à un seuil t1. Ainsi, les fréquences n*f1 et m*f2 peuvent être si proches que le maximum T du champ total (c'est-à-dire le champ sommé) CT dépasse les maxima des champs électriques C1 et C2 d'une quantité de. Une telle sélection des fréquences f1 et f2 est donc moins souhaitable, car l'effet recherché de réduction de l'énergie et/ou puissance moyenne ne peut être atteint ou est au moins affaibli.

La figure 6 montre schématiquement un deuxième exemple de relation entre des fréquences de pilotage choisies et une distribution spectrale d'énergie résultante du champ électrique (et donc de l'émission électromagnétique) provoqué par le dispositif émetteur selon des exemples de la présente divulgation.

Les diagrammes d4b et d5b de la figure 6 correspondent principalement aux diagrammes d4a et d5a de la figure 6. Toutefois, dans l'exemple de la figure 6, les fréquences n*f1 et m*f2 sont suffisamment éloignées pour que le maximum T du champ total (c'est-à-dire le champ additionné) CT ne dépasse pas (ou du moins pas sensiblement) les maxima des champs électriques C1 et C2. Une telle sélection des fréquences f1 et f2 est donc souhaitable, car l'effet recherché de réduction de l'énergie et/ou puissance moyenne peut être atteint.

En conséquence, la différence df entre les fréquences f1 et f2 peut dépasser le seuil minimal t1.

Par exemple, le seuil minimal t1 peut être choisi en fonction d'une distribution du champ électrique total, notamment de telle sorte que le maximum du champ électrique total ne dépasse pas les maxima des champs électriques liés aux fréquences f1, f2.

Donc, les fréquences de pilotage peuvent être suffisamment différentes et/ou éloignées l'une de l'autre de telle sorte que leur champ spectral additionné ne dépasse pas le champ spectral de l'une quelconque des fréquences de pilotage individuelles.

En conséquence, l'effet souhaité de réduction de la puissance et/ou énergie moyenne peut avantageusement être atteint.

Tous ces modes de réalisations et d'autres exemples tels que décrits ci-dessus sont donnés uniquement à titre d'exemple non limitatif, et peuvent être combinés et/ou modifiés selon la portée de la présente divulgation.

## Revendications

1. Procédé pour piloter un dispositif émetteur (20), dans lequel
le dispositif émetteur fonctionne avec une fréquences cible (f0),
le procédé comprenant :
piloter le dispositif émetteur (20) par un signal électrique comprenant différentes fréquences de pilotage (f1, f2) sélectionnées en fonction de la fréquence cible (f0).

2. Procédé selon la revendication 1, dans lequel au moins l'une des fréquences de pilotage (f1, f2) du signal électrique est différente de la fréquence cible (f0).

3. Procédé selon la revendication 1 ou 2, dans lequel les fréquences de pilotage (f1, f2) sont suffisamment différentes et/ou éloignées l'une de l'autre de telle sorte que : leur champ spectral additionné ne dépasse pas le champ spectral de l'une quelconque des fréquences de pilotage individuelles.

4. Procédé selon une des revendications précédentes, dans lequel les fréquences de pilotage (f1, f2) sont sélectionnées de telle sorte que la fréquence cible (f0) puisse être atteinte par une combinaison de la pluralité de fréquences de pilotage (f1, f2).

5. Procédé selon une des revendications précédentes, dans lequel le signal électrique est composé de formes d'onde successives (S1, S2, S3, S4) telles que chaque forme d'onde a pour fréquence l'une des fréquences de pilotage (f1, f2).

6. Procédé selon la revendication 5, dans lequel
les formes d'onde (S1, S2, S3, S4) sont répétées, et/ou
les formes d'onde (S1, S2, S3, S4) sont alternées entre elles, de sorte que chaque forme d'onde soit partiellement interrompue.

7. Procédé selon la revendication 5 ou 6, dans lequel
chaque forme d'onde (S1, S2, S3, S4) est pondérée par une valeur d'impédance et/ou de courant en fonction du signal électrique cible.

8. Procédé selon l'une quelconque des revendications 5 à 7, dans lequel
les formes d'onde forment une séquence temporelle prédéfinie de N1 pulses de fréquences de pilotage f1, suivi de N2 pulses de fréquences de pilotage f2, ..., suivi de Nn pulses de fréquences de pilotage fn,
N1, N2, ..., Nn étant des nombres entiers supérieurs ou égaux à 1.

9. Procédé selon l'une quelconque des revendications 5 à 8, dans lequel les formes d'onde forment une séquence temporelle aléatoire de pulses de fréquences de pilotage.

10. Procédé selon l'une quelconque des revendications 8 ou 9, dans lequel les formes d'onde et/ou les pulses de fréquences de pilotage sont configurés pour stimuler un élément piézoélectrique d'une sonde à ultrasons.

11. Procédé selon l'une quelconque des revendications 5 à 10, dans lequel le dispositif (20) est piloté par un signal électrique ayant la fréquence cible (f0), dans laquelle les formes d'onde sont sélectionnées, de telle sorte que leur ensemble puisse atteindre le signal électrique cible.

12. Procédé selon l'une quelconque des revendications 5 à 11, dans lequel les formes d'onde (S1, S2, S3, S4) sont sélectionnés de telle sorte qu'une énergie moyenne (qp), dite « Q-Peak », de chaque forme d'onde soit inférieure à l'énergie moyenne du signal électrique cible.

13. Procédé selon l'une quelconque des revendications précédentes, dans lequel le procédé est un procédé médical et/ou à ultrasons, et/ou le dispositif est une sonde médicale et/ou à ultrasons.

14. Procédé selon l'une quelconque des revendications précédentes, dans lequel le dispositif émetteur émet une onde pour traiter et/ou observer le milieu, et/ou le dispositif émet pour émettre une onde électro-magnétique lorsqu'il est piloté.

15. Programme d'ordinateur comprenant des instructions qui, lorsque le programme est exécuté par un ordinateur, conduisent celui-ci à mettre en oeuvre le procédé selon une des revendications précédentes.

16. Système (10) pour piloter un dispositif émetteur (20), dans lequel
le dispositif émetteur fonctionne avec une fréquences cible (f0),
le système étant configuré pour :
piloter le dispositif émetteur par un signal électrique comprenant différentes fréquences de pilotage (f1, f2) sélectionnées en fonction de la fréquence cible (f0).
